# EUROPEAN PATENT SPECIFICATION

(11) **EP 2 793 829 B1**
(45) Date of publication and mention of the grant of the patent: **12.09.2018**
(21) Application number: 12823027.3
(22) Date of filing: 20.12.2012
(51) Int. Cl.: A61K 8/43, A61K 8/44, A61Q 19/08

(54) **COMBINATION OF A NUCLEOPHILIC AGENT AND OF A NITROGEN-COMPRISING AGENT WITH A PKA OF GREATER THAN 11, FOR GLYCATED SKIN**
KOMBINATION EINES NUKLEOPHILEN WIRKSTOFFES UND EINES STICKSTOFFHALTIGEN WIRKSTOFFES MIT EINEM PKA ÜBER 11 FÜR GLYKIERTE HAUT
COMBINAISON D'UN AGENT NUCLÉOPHILE ET D'UN AGENT AZOTÉ À PKA SUPÉRIEUR À 11 POUR UNE PEAU EXPOSÉE À LA GLYCATION

(30) Priority: 22.12.2011 FR 1162285; 03.01.2012 US 201261582758 P
(43) Date of publication of application: 29.10.2014
(73) Proprietor: L'Oréal, 75008 Paris (FR)
(72) Inventor: SAMAIN, Henri, F-91570 Bievres (FR); BERNARD, Anne-Laure, F-92160 Antony (FR); KAUFFMANN, Myriam, F-94200 Ivry Sur Seine (FR)
(74) Representative: Nony
(86) International application number: PCT/IB2012/057528
(87) International publication number: WO 2013/093834

(56) References cited:
- EP-A1- 2 033 689
- WO-A1-93/04669
- WO-A1-95/33443
- JP-A- 62 249 908
- US-A- 5 852 009
- US-A- 5 939 078
- US-A1- 2004 198 795
- S.F. IGE ET AL: "The Role of Hyperglycemia in Skin Wrinkle Formation: Mediation of Advanced Glycation End-Products", RESEARCH JOURNAL OF MEDICAL SCIENCES, vol. 4, no. 5, 1 May 2010 (2010-05-01), pages 324-329, XP055041221, ISSN: 1815-9346, DOI: 10.3923/rjmsci.2010.324.329

## Description

The present invention relates to the cosmetic field of the treatment of the skin. It is targeted more particularly at restoring, at least in part, the original state, in particular in terms of suppleness, to skin which has been subjected to a phenomenon of glycation of the proteins, subsequently denoted under the expression "glycated skin".

Glycation is a nonenzymatic process, also known as nonenzymatic glycosylation, induced from reducing monosaccharides which can originate from inside the body (via the the lymphatic blood route or the interstitial medium) or from outside the body (exogenous sugars, dihydroxyacetone (DHA), indeed even pollutants) and accentuated in the presence of an oxidizing environment. More particularly, glycation involves the aldehyde or ketone functional groups of a monosaccharide (in particular glucose or ribose) which reacts according to the Maillard reaction with an amino group of an amino acid residue (such as, for example, lysine), generally an amino acid residue of a protein, to form a Schiff base. The latter, after an "Amadori" molecular rearrangement, can result, by a sequence of reactions, in bridgings, particularly intramolecular bridgings, such as, for example, of pentosidine type. This results in the formation of a succession of glycation products which are also subject to oxidative stresses and the content of which will steadily increase as a function of age.

The glycation products are, for example, pyrraline, carboxymethyl-lysine (CML), pentosidine, crossline, N^{ε}-(2-carboxyethyl)lysine (CEL), glyoxal-lysine dimer (GOLD), methylglyoxal-lysine dimer (MOLD), 3DG-ARG imidazolone, versperlysines A, B and C, threosidine or advanced glycation endproducts (or AGEs), which are described as a heterogeneous class of several hundred molecules.

It should be noted that glycation occurs at extra- or intracellular protein fibers, such as intracellular collagen, elastin, fibronectin, laminin or vimentin fibers, and the accumulation of the AGEs occurs in particular at the proteins having a slow rate of replacement (or having a low turnover), such as collagen fibers. This is because the glycation of collagen steadily increases with age, resulting in a steady increase in the content of glycation products in the skin.

The phenomenon of glycation thus results in changes in the state and capabilities of the skin, vascular walls, crystallin and organs rich in structural proteins, in particular in terms of mechanical properties (tactile properties for the skin) and of visual appearance or in physiological terms, such as the neurological or healing capabilities, these detrimental changes being well known in the case of poorly equilibrated diabetic subjects.

Thus, during aging of the skin, the physicochemical properties of the collagen become modified and the latter becomes more difficult to dissolve and more difficult to degrade. This results in a stiffening of the tissues, essentially leading to a loss in elasticity and in suppleness of the skin. In addition, these phenomena are enhanced by exposure to UV radiation. The glycation of protein fibers plays a role in the phenomenon of photoaging [1].

Glycation is also involved in the "orange peel" appearance characteristic of cellulite. This is because, in cellulite, glycation of the collagen constituting the greater part of the connective framework leads to a stiffening of the tissues, which then trap fat globules. The skin thus exhibits a sequence of bumps, formed from fat clusters, and of hollows, formed from stiffened connective framework, which are characteristic of the "orange peel" appearance.

Glycation can also result in a change in color of the skin, which will reinforce the aged appearance of glycated skin.

The phenomenon of glycation can be more particularly exacerbated in people having or having had attacks of hyperglycemia or in people having poorly regulated glycemia, as in the case of poorly equilibrated diabetics. This is all the more problematic when it concerns glycation of proteins having a low rate of replacement.

A first approach for overcoming the effects of glycation consists in developing products capable of reducing, indeed even inhibiting, the phenomenon of glycation of proteins ("antiglycation" concept). Mention may be made, as examples of compounds proposed for their ability to inhibit the glycation reaction, of aminoguanidine, taurine, some vitamins (B1, B6), thiazolium derivatives or ascorbic acid.

Mention may also be made of the use of N-acetylhydroxyproline for preventing or reducing glycation in fibroblasts (WO 2008/101692).

However, this "antiglycation" route requires that the skin be treated regularly and does not make it possible to treat skin which is already glycated.

The present invention is targeted at another approach, that described as "deglycation". In other words, it concerns the development of treatments capable of cleaving the glycation bonds of glycated proteins

The "deglycation" of glycated proteins has already been explored, in particular by Germayel *et al.,* who describe a process of deglycation by enzymatic phosphation. It appears to bring about destabilization and detachment of the sugar ([2], [3]). In addition, Szwergold *et al.* ([4], [5], [6]) describe the treatment of glycated proteins with a nucleophile, such as a glutathione, in order to detach the sugar and to release the amine of the protein. Mention may also be made of Ahmad *et al.* [7], who propose allylcysteine for deglycating proteins. However, the studies which have been carried out have shown that the use of just one nucleophile, such as, for example, cysteine, does not modify, or not significantly, the appearance of glycated skin.

Furthermore, in order to be suitable for the treatment of glycated skin, a deglycation system has to satisfy a number of requirements. Thus, it should not, or not significantly, cleave peptide bonds, which guarantee the integrity, firmness and plasticity of the skin. Furthermore, it should not create a major detrimental change in the skin exfoliation cutaneous barrier; in particular, it should not detrimentally affect intercellular linkages (corneosomes). Finally, bringing such a system into contact with the skin to be treated must not bring about additional problems, such as redness or irritation.

The present invention is aimed specifically at providing a novel deglycation system which makes it possible to treat signs of aging of the skin related to the glycation phenomenon, in particular to restore, at least in part, the original appearance and capabilities of glycated skin (suppleness, color, sensitivity and healing power), and which makes it possible more particularly to render supple skin which has been hardened by glycation.

Thus, the invention relates, according to a first of its aspects, to the cosmetic use of a combination of a least one nucleophilic agent and of a least one nitrogen-comprising agent with a pKa of greater than 11, distinct from said nucleophilic agent, for treating signs of aging and photoaging of the skin related to glycation.

According to a specific embodiment, said nucleophilic agent and said nitrogen-comprising agent with a pKa of greater than 11 are employed in one or more compositions exhibiting a pH ranging from 5 to 13, preferably from 7 to 12, especially from 7 to 11, in particular from 8 to 10 and more particularly from 8 to 9.5.

According to a specific embodiment, the present invention employs at least one thiolate derivative and at least one guanidinium ion or one of its derivatives.

Surprisingly, the inventors have discovered that such a combination of active agents advantageously makes it possible to effectively act against signs of aging and photoaging of the skin, in particular, as illustrated in example 1, to restore, at least in part, the original suppleness of glycated skin.

Furthermore, the combination according to the invention does not bring about, or not significantly, cleavage of peptide bonds. It is thus possible, by employing the combination of active agents according to the invention, to effectively treat glycated skin, in particular to render it supple, without affecting the structure of the skin.

Thiol derivatives, such as, for example, cysteine, are known for their property as reducing agents for disulfide bridges. They are thus widely used for permanent waves for the hair.

With regard to guanidine, it has already been provided in cosmetics for many applications on the skin, for example as antiwrinkle agent (WO 98/15260).

However, to the knowledge of the Applicant Company, the combination of a nucleophile and of a nitrogen-comprising agent with a pKa of greater than 11 according to the invention, in particular the combination of a thiolate derivative, such as cysteine, and of a guanidinium ion or one of its derivatives, has never yet been proposed for the treatment of glycated skin, in particular for rendering supple skin hardened by glycation.

The term "the skin" is understood to mean the entire covering of the body, including the lips, scalp and mucous membranes with their appendages.

The expression "signs of aging and photoaging of the skin related to glycation" is understood to denote all of the signs characteristic of glycated skin and more particularly a loss in suppleness of the skin, in other words a hardening of the skin, a change in color of the skin, which darkens, the elastosic appearance and the "orange peel" appearance of the skin discussed above.

In particular, the signs of aging of the skin related to glycation are distinct from the wrinkles.

Thus, according to one of its aspects, the present invention relates to the use of a combination of active agents as described above for rendering glycated skin supple, more particularly skin hardened as a result of glycation.

It is also targeted at the use of such a combination for treating the elastosic appearance of skin which has been subjected, as time goes by, to lengthy periods of exposure to sunlight, in particular on easily damaged areas, such as the neck, nape and neckline.

It is also targeted at the use of such a combination for treating the "orange peel" appearance of glycated skin.

According to a specific embodiment, the present invention relates to the use of the combination of at least one thiolate derivative, in particular cysteine or one of its derivatives, and of at least one guanidinium ion or one of its derivatives for rendering the skin supple.

According to another of its aspects, the invention relates to a method for the cosmetic treatment of glycated skin, in particular to render glycated skin supple, comprising at least bringing the skin to be treated into contact with a combination of active agents as is defined above.

The method of the invention is more particularly intended for skin which has glycated gradually during life, in particular the skin of elderly people, people having or having had attacks of hyperglycemia or problems of deficiency of the natural "antiglycation" protective systems, resulting in a phenomenon of accentuated glycation, or for thin and easily damaged areas exposed to sunlight in a repeated and prolonged way, such as the neck, nape or neckline, and having undergone actinic elastosis.

The combination under consideration according to the invention can be employed with one or more additional compounds, chosen in particular from pH regulating agents, chaotropic agents, additional reducing agents, simple amines or polyamines, desquamating agents, dermo-relaxing agents, anti-inflammatory agents, agents which stimulate renewal of tissue and extracellular matrices, or antioxidants.

As expanded upon more particularly below, bringing the skin to be treated into contact with the combination of active agents according to the invention is understood to mean more particularly the application, simple or in combination with a system which facilitates the penetration, and/or the injection into the thickness of the skin of one or more compositions comprising said nucleophilic agent and/or said agent with a pKa of greater than 11.

Other characteristics, alternative forms and advantages of the employment of the combination according to the invention will emerge more clearly from reading the description, examples and figures which will follow, given by way of illustration and without implied limitation.

In the continuation of the text, the expressions "between ... and ...", "ranging from ... to ..." and "varying from ... to ..." are equivalent and are intended to mean that the limits are included, unless otherwise mentioned.

Unless otherwise indicated, the expression "comprising a" should be understood as "comprising at least one".

### NUCLEOPHILIC AGENT

As touched on above, the present invention employs, according to a first of its aspects, at least one nucleophilic agent.

The term "nucleophilic agent" is intended to denote a compound comprising at least one nucleophilic chemical functional group, that is to say a functional group attracted by electrophilic entities. The term "electrophile" is understood to mean a charged or partially charged entity.

A nucleophile reacts by donating electrons to the electrophilic compound.

The nucleophilic agent can be a molecule or an ion which has a non-bonding electron pair. It may or may not be negatively charged.

The nucleophilic agent can thus react with an electrophilic entity in order to create a new covalent bond or else to produce an electrostatic bond.

According to a specific embodiment, the nucleophilic agent is employed in a medium with a pH ranging from 5 to 13, especially from 7 to 12 and in particular from 8 to 10. Preferably, the medium is an aqueous medium, as described more specifically subsequently.

Said nucleophilic agent can more particularly be chosen from compounds having a nucleophilic functional group chosen from the following functional groups:
- thiolate (RS⁻);
- halide, in particular iodide (I⁻), bromide (Br⁻), chloride (Cl⁻) or fluoride (F⁻), and oxidized halide functional groups, such as, for example, hypoiodite (IO⁻);
- cyanate (OCN⁻) and its derivatives, such as, for example, isocyanate or isothiocyanate;
- sulfite (SO₃²⁻) and other oxidized sulfur functional groups, such as, for example, bisulfite or hydrogen sulfite (HSO₃⁻), thiosulfate (S₂O₃²⁻) and tetrathionate (S₄O₆²⁻); and
- phosphite (HPO₃²⁻) or phosphine and its derivatives, in particular triarylphosphine, trialkylphosphine, such as, for example, phosphinetripropionic triacid, ou triphenylphosphine.

The nucleophilic agent can be chosen from plant or biotechnological extracts or their derivatives comprising these functional groups, such as, for example, coleus extracts.

According to a particularly preferred embodiment, said nucleophilic agent is chosen from thiol derivatives.

Preferably, said nucleophilic agent exhibits a negatively charged thiol functional group, also known as thiolate (RS⁻) functional group.

It is understood that the charged or uncharged form of said nucleophilic agent will depend on the pH of the medium in which it is employed.

In particular, said thiol derivative can be chosen from:
- cysteine or one of its N-alkylated, N-acylated or C-alkylated derivatives; homocysteine and its N-alkylated, N-acylated or C-alkylated derivatives; and peptides comprising them, such as, for example, reduced glutathione;
- cysteamine or its N-alkylated or N-acylated derivatives;
- thioglycolic acid or one of its derivatives, thiolactic acid or one of its derivatives, or mercaptopropionic acid or one of its derivatives.

Preferably, said nucleophilic agent exhibits a limited reducing power.

According to a particularly preferred embodiment, said nucleophilic agent is chosen from cysteine or one of its N-alkylated, N-acylated or C-alkylated derivatives; homocysteine and its N-alkylated, N-acylated or C-alkylated derivatives; and peptides comprising them, such as, for example, reduced glutathione, these compounds exhibiting in particular a negatively charged thiol functional group.

Preferably, said nucleophilic agent is chosen from cysteine or from one of its N-alkylated, N-acylated and C-alkylated derivatives, in particular exhibiting a negatively charged thiol functional group.

In particular, said nucleophilic agent can be chosen from L-cysteine and N-acetyl-L-cysteine, in particular exhibiting a negatively charged thiol functional group.

Preferably, said nucleophilic agent is N-acetyl-L-cysteine, in particular exhibiting a negatively charged thiol functional group.

According to another specific embodiment, said nucleophilic agent is chosen from compounds having an isocyanate functional group.

According to yet another specific embodiment, said nucleophilic agent is chosen from compounds having a halide functional group.

### NITROGEN-COMPRISING AGENT WITH A pKA OF GREATER THAN 11

As specified above, the present invention employs, according to another of its aspects, at least one nitrogen-comprising agent with a pKa of greater than 11.

Natural marine or plant or biotechnological extracts or their derivatives may be concerned.

Preferably, said nitrogen-comprising agent exhibits a pKa of greater than 12.

According to a specific embodiment, said agent with a pKa of greater than 11 can be provided in a positively charged form.

It is understood that the charged or uncharged form of said agent with a pKa of greater than 11 will depend on the pH of the medium in which it is employed.

According to a particularly preferred embodiment, said agent with a pKa of greater than 11 is employed in a medium with a pH ranging from 5 to 13, preferably from 7 to 12, especially from 7 to 11, in particular from 8 to 10 and more particularly from 8 to 9.5. Preferably, the medium is an aqueous medium, as described more specifically subsequently.

According to a specific embodiment, said agent with a pKa of greater than 11 is chosen from nitrogen-comprising agents.

It can more particularly be chosen from guanidine or one of its derivatives; imidazole or a compound comprising an imidazolyl group; or pyrrole or a compound comprising a pyrrolyl group.

Preferably, said nitrogen-comprising agent is chosen from guanidine and its derivatives, preferably exhibiting a positively charged guanidine functional group.

Said nitrogen-comprising agent according to the invention can thus be a compound of formula:

NHR=C(NR'R"₂)⁺

in which R, R' and R" are chosen, independently of one another, from a hydrogen and an alkyl group having from 1 to 6 carbon atoms.

Preferably, said agent with a pKa of greater than 11 is the guanidinium ion (R, R' and R" represent hydrogen atoms), more particularly employed in the form of guanidine carbonate, of guanidine hydroxide or of guanidine thiocyanate.

According to a specific embodiment, said nucleophilic agent is a thiol derivative, chosen in particular from cysteine and its N-alkylated, N-acylated and C-alkylated derivatives, preferably exhibiting a negatively charged thiol derivative, and said agent with a pKa of greater than 11 is guanidine or one of its derivatives, preferably exhibiting a positively charged guanidine functional group.

Preferably, the invention employees N-acetyl-L-cysteine and the guanidinium ion, in particular in the form of guanidine carbonate.

According to a specific embodiment, said nucleophilic agent and said agent with a pKa of greater than 11, in particular as defined above, are employed in a nucleophilic agent/agent with a pKa of greater than 11 molar ratio ranging from 10 to 1, in particular from 5 to 1 and more particularly of approximately 1.

Thus, according to a specific embodiment, the invention employs a thiol derivative, in particular as defined above, and a guanidinium ion or one of its derivatives in a thiolate derivative/guanidinium ion molar ratio ranging from 10 to 1, in particular from 5 to 1 and more preferably of approximately 1.

### METHOD

As mentioned above, a further subject matter of the invention, according to another of its aspects, is a cosmetic method for treating signs of aging of the skin related to glycation, in particular intended to render glycated skin supple, comprising at least the stages consisting in:
(i) bringing the skin to be treated into contact with at least one nucleophilic agent, in particular a thiol derivative, in particular exhibiting a negatively charged thiol functional group; and
(ii) bringing the skin to be treated into contact with at least one nitrogen-comprising agent with a pKa of greater than 11, distinct from said nucleophilic agent, in particular a guanidinium ion or one of its derivatives, it being possible for stages (i) and (ii) to be carried out consecutively in the chronological order (i) and then (ii), or in the reverse order (ii) and then (i), or simultaneously.

According to a first alternative form of the method of the invention, stages (i) and (ii) are carried out consecutively in this order or in the reverse order.

In the context of this alternative embodiment, said nucleophilic agent and said agent with a pKa of greater than 11 are formulated in two separate compositions.

According to another alternative form of the method of the invention, stages (i) and (ii) are carried out simultaneously, said nucleophilic agent and said agent with a pKa of greater than 11 being formulated within one and the same composition.

Preferably, the composition is obtained, prior to being brought into contact with the skin, by extemporaneous mixing of at least one composition comprising said nucleophilic agent and of at least one composition comprising said agent with a pKa of greater than 11.

Thus, according to yet another of its aspects, the present invention relates to a kit comprising, in separate containers, at least one nucleophilic agent and at least one nitrogen-comprising agent with a pKa of greater than 11, in particular as defined above.

Said kit additionally can comprise at least one means for bringing the skin into contact with said active agents, in particular a means for application to or injection into the thickness of the skin, as described below.

Thus, according to a preferred embodiment, the present invention relates to a cosmetic kit comprising, in separate containers, at least one first composition comprising a nucleophilic agent, at least one second composition comprising a nitrogen-comprising agent with a pKa of greater than 11, in particular as defined above, and at least one means for application of a composition to the skin or one means of injection of a composition into the thickness of the skin.

According to another specific embodiment, said kit can also comprise, in a separate container, an additional composition comprising one or more supplementary active agents, in particular as defined subsequently.

According to a first alternative embodiment, the operation in which said active agents are brought into contact with the skin to be treated can comprise, indeed can even consist of, the application to the skin of said composition or compositions comprising said nucleophilic agent and/or said agent with a pKa of greater than 11.

Said composition or compositions can be applied using the palm of the hand or the fingers, or else by conventional application means, such as, for example, using a sponge or a brush, a patch or an iontophoretic system.

According to another alternative form of the method of the invention, the operation in which said active agents are brought into contact with the skin to be treated can comprise, indeed can even consist of, the injection into the thickness of the skin of one or more compositions comprising said nucleophilic agent and/or said agent with a pKa of greater than 11.

It is understood that the injection according to the invention is restricted to a superficial region. It is more particularly an intraepidermal and/or intradermal injection.

Mention may be made, among the injection means suitable for an intraepidermal or intradermal injection, for example, of a syringe, microneedle rollers or patches, or the needles normally used for mesotherapy, also known as mesolift or mesoglow when it is carried out on the face.

Of course, the method of the invention can combine these two alternative embodiments. In other words, the method of the invention can comprise the application to and/or the injection into the thickness of the skin of one or more compositions comprising said nucleophilic agent and/or said agent with a pKa of greater than 11.

According to a specific embodiment, the operation in which the skin is brought into contact with said nucleophilic agent and/or said agent with a pKa of greater than 11 according to the invention can be accompanied by the employment of one or more means normally employed for promoting the penetration of said agent or agents into the skin.

For example, the method can additionally comprise the employment, in said composition or compositions comprising said nucleophilic agent and/or said agent with a pKa of greater than 11 according to the invention and/or in an additional composition, of compounds which promote the penetration into the skin, such as a solvent, a surfactant or a hydrotrope, for example benzyl alcohol or propylene carbonate.

According to another specific embodiment, the skin to be treated can be subjected, at least in part, prior to, simultaneously with or subsequent to the operation in which it is brought into contact with said active agents, to a mechanical action, such as a micropeeling or a controlled chemical peeling, to an energetic action, such as the application of heat, of electric currents or of electromagnetic waves, submission to microwaves, laser, ultrasound, and the like.

According to a further specific embodiment, it is possible to create a mechanical tension on the skin to be treated, prior to, simultaneously with or subsequent to bringing it into contact with said active agents.

### COMPOSITIONS

Said composition or compositions comprising said nucleophilic agent and/or said agent with a pKa of greater than 11 comprise a physiologically acceptable medium.

The term "physiologically acceptable medium" is understood to mean a medium devoid of toxicity and compatible with the application and the penetration, or the injection into the thickness of the skin, of the composition.

According to a specific embodiment, said composition or compositions, in particular when they are intended for injection into the thickness of the skin, are sterile. The term "sterile" is intended to describe an environment devoid of microbial contamination capable of guaranteeing that the compound and/or the composition in which it is present has the safety required for administration in the skin, in particular intraepidermal and/or intradermal administration. In particular, it is essential for the composition formed of the physiologically acceptable medium comprising said compound and which has to be administered according to an injection technique to be devoid of any impurity or contaminant of microbial origin capable of initiating an adverse side reaction in the host organism.

The physiologically acceptable medium is more particularly an aqueous medium. The aqueous medium can be formed of water and/or of one or more water-miscible organic solvents, such as, for example, chosen from C₁₋₈ monoalcohols, in particular C₁₋₅ monoalcohols. Preferably, the aqueous medium is constituted of water.

A composition according to the invention can comprise water in a content ranging from 0% to 98% by weight and more particularly from 50% to 90% by weight, with respect to its total weight.

The physiologically acceptable medium in the case of microinjection can be isotonic with serum.

The concentration of nucleophilic agent and/or agent with a pKa of greater than 11 in said composition(s) can be between 0.01 and 5 mol/l, in particular between 0.05 and 1 mol/l.

In particular, the contents of nucleophilic agent and of agent with a pKa of greater than 11 in said composition or compositions are such that a mixture of the compositions comprises said nucleophilic agent and said agent with a pKa of greater than 11 in a molar ratio ranging from 10 to 1, in particular from 5 to 1 and more particularly of approximately 1.

As mentioned above, said composition or compositions more particularly exhibit a pH ranging from 5 to 13, preferably from 7 to 12, in especially from 7 to 11, in particular from 8 to 10 and more particularly from 8 to 9.5.

The pH of said composition(s) can be adjusted by addition of a pH regulating agent, chosen in particular from citric acid and sodium hydroxide.

As described below, the pH regulating agent can be employed in said composition or compositions comprising said nucleophilic agent and/or said agent with a pKa of greater than 11, and/or in an additional composition.

### Supplementary active agents

According to a specific embodiment, the invention can employ, in addition to said nucleophilic agent and said agent with a pKa of greater than 11, one or more supplementary cosmetic active agents.

They can more particularly be active agents chosen from:
- chaotropic agents, such as, for example, urea;
- additional reducing agents, such as, for example, ascorbic acid;
- amines or polyamines;
- desquamating agents, such as urea; β-hydroxy acids (BHAs), for example salicylic acid and its derivatives, other than 5-(n-octanoyl)salicylic acid; α-hydroxy acids (AHAs), glycolic, citric, lactic, tartaric, malic or mandelic acid; 4-(2-hydroxyethyl)piperazine-1-propanesulfonic acid (HEPES); Sophora japonica extract; honeys; N-acetylglucosamine; sodium methylglycine diacetate, or plant extracts comprising it, such as the willow leaf extract;
- dermo-relaxing agents, for example manganese gluconate, wild yam, rock samphire, glycine and alverine;
- anti-inflammatory agents, such as licorice derivatives and glycerrhetinic acid, Aloe vera, Aloe ferox, extracts of yeasts, such as Saccharomyces cerevisiae, extracts of rose and rosemary, of camomile, of common comfrey, allantoin, D-panthenol, azulene and chamazulene;
- agents which stimulate renewal of tissue and extracellular matrices, such as Centella asiatica extracts, fractions of hyaluronic acids, alginates and chitosans;
- antioxidants, in particular plant polyphenols;
and their mixtures.

These supplementary active agents can be employed in said composition or compositions comprising said nucleophilic agent and/or said agent with a pKa of greater than 11, and/or in an additional composition.

According to a specific embodiment, the method of the invention can comprise the stages consisting in:
(a) bringing the skin to be treated into contact with at least said nucleophilic agent and at least said agent with a pKa of greater than 11, for example by application of a composition comprising said agents; and
(b) bringing the skin to be treated into contact with one or more supplementary active agents, in particular by application of an additional composition comprising one or more supplementary active agents.

These supplementary active agents can be present in said composition or compositions in a content ranging from 0.001% to 50% by weight, preferably from 0.01% to 10% by weight and more particularly from 0.01% to 5% by weight, with respect to the total weight of the composition.

Of course, a person skilled in the art will take care to choose said optional additional active agent or agents and/or their amount so that the advantageous properties of the combination of active agents according to the invention are not, or not substantially, detrimentally affected by the envisioned addition.

Of course, said composition or compositions of the invention can comprise any other adjuvant normal in the cosmetic field, such as hydrophilic or lipophilic gelling agents, hydrophilic or lipophilic active agents, preservatives, solvents, fragrances, fillers, screening agents, pigments, odor absorbers, sequestering agents and colorants. The amounts of these various adjuvants are those conventionally used in the field under consideration, for example from 0.01% to 20% of the total weight of the composition. In any case, these adjuvants and their proportions will be chosen so as not to harm the properties desired for the combination of active agents according to the invention.

Said composition or compositions according to the invention can be provided in any formulation form normally used in the cosmetic and dermatological fields.

They can be more or less fluid and can have the appearance of a liquid, a white or colored cream, an ointment, a milk, a lotion, a serum, a paste or a foam. It can optionally be applied to the skin in aerosol form. They can also be provided in solid form, in particular in stick form.

They can also be provided in the form of a patch, of a dressing or of an iontophoretic system.

Of course, it is up to a person skilled in the art to choose the appropriate formulation form and its method of preparation on the basis of his general knowledge, in particular from the viewpoint of the embodiment envisioned, according to the regions to be treated and according to whether the composition will be applied to the skin to be treated or injected into the thickness of the skin.

The examples which follow are presented by way of illustration and without implied limitation of the invention.

### EXAMPLE

### Demonstration of the effectiveness of the combination of the invention

The following compositions were prepared (the percentages shown are percentages by weight).

**Table 1**

| | **Formula A (outside the invention)** | **Formula B (outside the invention)** | **Formula C (according to the invention)** | **Formula D (according to the invention)** |
|---|---|---|---|---|
| **L-Cysteine**⁽¹⁾ | - | 12 | 12 | - |
| **N-Acetyl-L-cysteine**⁽²⁾ | - | - | - | 16.5 |
| **Guanidine carbonate**⁽³⁾ | 17.5 | - | 17.5 | 17.5 |
| **Citric acid** | q.s. pH 9 | - | - | - |
| **Sodium hydroxide** | - | q.s. pH 9 | - | - |
| **Water** | q.s. 100% | q.s. 100% | q.s. 100% | q.s. 100% |
| **pH Value** | 9 | 9 | 9 | 9 |

| | | | | |
|---|---|---|---|---|
| ⁽¹⁾ sold by Ajinomoto, ⁽²⁾ sold by Nippon Rika, ⁽³⁾ sold by Palmer Company. | | | | |

### Procedure

The guanidine is first of all dissolved in the water and then the cysteine derivative or the citric acid is added.

The solution obtained is transparent.

### Evaluations of the formulas obtained

### Protocol for testing on a glycated skin model

The skin used is that of a piece of nondermatomed pig "back bacon". The piece comprises the epidermis, the dermis, the hypodermis and a few residual muscle fibers. The test is carried out on the epidermis side.

The skin is immersed for 24 h in a solution of methylglyoxal (10% by weight) in water and is then copiously rinsed with Millipore water before being immersed for 20 minutes in one of the solutions described above.

The measurement of hardness is carried out with a TAXT2iHR texture analyser in Pa. A force of 100 g is applied, which force corresponds to the force which would be generated by feeling one's face with one's fingers with a rate of 0.1 mm/s with a rod with a cross-section of 6 mm. The rod is made to penetrate to a depth of 0.5 mm.

### Results

The measurement results are collated in table 2 below.

**Table 2**

| **Samples** | **1 (outside the invention)** | **2 (outside the invention)** | **3 (according to the invention)** | **4 (according to the invention)** |
|---|---|---|---|---|
| **Force in grams** | | | | |
| **Before glycation** | 24 ± 4 | 24 ± 4 | 18 ± 2 | 23 ± 6 |
| **After glycation** | 240 ± 59 | 240 ± 59 | 261 ± 79 | 327 ± 54 |
| **After treatment** | + formula A | + formula B | + formula C | + formula D |
| | 175 ± 26 | 175 ± 34 | 133 ± 21 | 47 ± 25 |
| **Rendering supple (after treatment/before treatment)** | 1.3 (+27%) | 1.3 | 2 (+50%) | 7 (+85%) |

The treatment with methylglyoxal makes it possible to generate hardening of the skin by a factor of 10.

The formulas A and B not in accordance with the invention bring about a slight effect in rendering the skin supple, whereas the formulas C and D according to the invention bring about a significantly improved effect in rendering the skin supple. The acetylcysteine/guanidine combination makes it possible to restore a suppleness close to the original suppleness of the skin.

### References

[1] Jeanmaire et al., Glycation during human intrinsic and actinic ageing; an in vivo and in vitro study. Br. J. Dermatol., 2001, 145, 10-8;
[2] Gemayel et al., "Many fructosamine 3-kinase homologues in bacteria are ribulosamine/erythrulosamine 3-kinases potentially involved in protein deglycation", FEBS J 274:4360-74;
[3] Fortpied et al., "Magnesium-dependent phosphatase-1 is a protein-fructosamine-6-phosphatase potentially involved in glycation repair", J. Biol. Chem., 281, 18378-85;
[4] Szwergold et al., Ann. NY Acad. of Science, 1043, 845-864 (2005);
[5] Szwergold et al., "Alpha-thiolamines such as cysteine and cysteamine act as effective transglycating agents due to formation of irreversible thiazolidine derivatives", Med. Hypotheses, 66, 698-707;
[6] Ahmad et al., "Aged garlic extract and S-allyl cysteine prevent formation of advanced glycation endproducts", N. Eur. J. Pharmacol., 2007 Apr 30;561(1-3):32-8;
[7] Szwergold et al., "Intrinsic toxicity of glucose, due to non-enzymatic glycation, is controlled in-vivo by deglycation systems including: FN3K-mediated deglycation of fructosamines and transglycation of aldosamines", Med. Hypotheses, 65: 337-48.

## Claims

1. The cosmetic use of a combination of a least one nucleophilic agent and of a least one nitrogen-comprising agent with a pKa of greater than 11, distinct from said nucleophilic agent, for treating signs of aging and photoaging of the skin related to glycation, the said signs being chosen in a group comprising a loss in suppleness of the skin, a change of color of the skin, an elastosic appearance of the skin and an orange peel appearance of the skin.

2. The cosmetic use as claimed in claim 1 for rendering glycated skin supple.

3. The use as claimed in either one of the preceding claims, wherein said nucleophilic agent is chosen from compounds having a nucleophilic functional group chosen from the following functional groups:
- thiolate;
- halide, in particular iodide, bromide, chloride or fluoride, and oxidized halide functional groups, such as, for example, hypoiodite;
- cyanate and its derivatives, such as, for example, isocyanate or isothiocyanate;
- sulfite and other oxidized sulfur functional groups, such as, for example, bisulfite or hydrogen sulfite, thiosulfate and tetrathionate; and
- phosphite or phosphine and its derivatives, in particular triarylphosphine, trialkylphosphine, such as, for example, phosphinetripropionic triacid, ou triphenylphosphine.

4. The use as claimed in any one of the preceding claims, wherein said nucleophilic agent is a thiol derivative chosen in particular from:
- cysteine and its N-alkylated, N-acylated or C-alkylated derivatives; homocysteine and its N-alkylated, N-acylated or C-alkylated derivatives; and peptides comprising them, such as, for example, reduced glutathione;
- cysteamine or its N-alkylated or N-acylated derivatives;
- thioglycolic acid or one of its derivatives, thiolactic acid or one of its derivatives, or mercaptopropionic acid or one of its derivatives.

5. The use as claimed in any one of the preceding claims, wherein said nucleophilic agent is chosen from cysteine or one of its N-alkylated, N-acylated or C-alkylated derivatives; homocysteine and its N-alkylated, N-acylated or C-alkylated derivatives; and peptides comprising them, such as, for example, reduced glutathione.

6. The use as claimed in any one of the preceding claims, wherein said agent with a pKa of greater than 11 is chosen from guanidine or one of its derivatives; imidazole or a compound comprising an imidazolyl group; or pyrrole or a compound comprising a pyrrolyl group.

7. The use as claimed in any one of the preceding claims, wherein said agent with a pKa of greater than 11 is chosen from guanidine and its derivatives.

8. The use as claimed in any one of the preceding claims, wherein said agent with a pKa of greater than 11 is the guanidinium ion, more particularly employed in the form of guanidine carbonate, of guanidine hydroxide or of guanidine thiocyanate.

9. The use as claimed in any one of the preceding claims, wherein said nucleophilic agent is a thiol derivative, chosen in particular from cysteine and its N-alkylated, N-acylated and C-alkylated derivatives, and said agent with a pKa of greater than 11 is guanidine or one of its derivatives.

10. The use as claimed in any one of the preceding claims, wherein said nucleophilic agent is N-acetyl-L-cysteine and said agent with a pKa of greater than 11 is the guanidinium ion, in particular in the form of guanidine carbonate.

11. The use as claimed in any one of the preceding claims, wherein said nucleophilic agent and said agent with a pKa of greater than 11 are employed in a nucleophilic agent/agent with a pKa of greater than 11 molar ratio ranging from 10 to 1, in particular from 5 to 1 and more particularly of approximately 1.

12. A cosmetic method for treating signs of aging of the skin related to glycation, in particular intended to render glycated skin supple, comprising at least the stages consisting in:
(i) bringing the skin to be treated into contact with at least one nucleophilic agent; and
(ii) bringing the skin to be treated into contact with at least one nitrogen-comprising agent with a pKa of greater than 11, distinct from said nucleophilic agent;
it being possible for stages (i) and (ii) to be carried out consecutively in the chronological order (i) and then (ii), or in the reverse order (ii) and then (i), or simultaneously, and the said signs being chosen in a group comprising a loss in elasticity of the skin, a loss in suppleness of the skin, a change of color of the skin, an elastosic appearance of the skin and an orange peel appearance of the skin.

13. The method as claimed in claim 12, **characterized in that** it comprises bringing into contact with the skin a composition obtained by extemporaneous mixing of at least one composition comprising said nucleophilic agent and of at least one composition comprising said agent with a pKa of greater than 11.

14. The method as claimed in claim 12 or 13, **characterized in that** it comprises the application to and/or the injection into the thickness of the skin of one or more compositions comprising said nucleophilic agent and/or said agent with a pKa of greater than 11.

15. The method as claimed in any one of claims 12 to 14, **characterized in that** said nucleophilic agent and said agent with a pKa of greater than 11 are defined as claimed in any one of claims 3 to 11.

16. The method as claimed in any one of claims 12 to 15, wherein the operation wherein the skin is brought into contact with said nucleophilic agent and/or said agent with a pKa of greater than 11 is accompanied by the employment of one or more means for promoting the penetration of said agent or agents into the skin, in particular compounds which promote the penetration into the skin, such as a solvent, surfactant or hydrotrope, a mechanical action, such as a micropeeling, or an energetic action, such as the application of heat, of electric currents, of electromagnetic waves, of microwaves or of ultrasound.

17. A cosmetic kit comprising, in separate containers, at least one first composition comprising a nucleophilic agent, at least one second composition comprising a nitrogen-comprising agent with a pKa of greater than 11, in particular defined as claimed in any one of claims 3 to 11, and at least one means for application of a composition to the skin or one means of injection of a composition into the thickness of the skin.

## Patentansprüche

1. Kosmetische Verwendung einer Kombination von mindestens einem nukleophilen Wirkstoff und mindestens einem stickstoffhaltigen Wirkstoff mit einem pKa über 11, der verschieden von dem nukleophilen Wirkstoff ist, zum Behandeln von Anzeichen von Alterung und Lichtalterung der Haut im Zusammenhang mit Glykierung, wobei die Anzeichen aus einer Gruppe ausgewählt sind, welche einen Verlust von Geschmeidigkeit der Haut, eine Farbänderung der Haut, ein Elastose-Phänomen der Haut und ein Orangenhaut-Phänomen der Haut umfasst.

2. Kosmetische Verwendung wie in Anspruch 1 beansprucht zum Wiederherstellen von Geschmeidigkeit von glykierter Haut.

3. Verwendung wie in einem der vorhergehenden Ansprüche beansprucht, wobei der nukleophile Wirkstoff aus Verbindungen mit einer nukleophilen funktionellen Gruppe ausgewählt ist, die aus den folgenden funktionellen Gruppen ausgewählt ist:
- Thiolat;
- Halogenid, insbesondere Iodid, Bromid, Chlorid oder Fluorid, und oxidierten funktionellen Halogenidresten, wie zum Beispiel Hypoiodit;
- Cyanat und seinen Derivaten, wie zum Beispiel Isocyanat oder Isothiocyanat;
- Sulfit und weiteren oxidierten funktionellen Schwefelresten, wie zum Beispiel Bisulfit oder Hydrogensulfit, Thiosulfat und Tetrathionat; und
- Phosphit oder Phosphin und ihren Derivaten, insbesondere Triarylphosphin, Trialkylphosphin, wie zum Beispiel Phosphintripropiontrisäure oder Triphenylphosphin.

4. Verwendung wie in einem der vorhergehenden Ansprüche beansprucht, wobei der nukleophile Wirkstoff ein Thiolderivat ist, welches insbesondere ausgewählt ist aus:
- Cystein und seinen N-alkylierten, N-acylierten oder C-alkylierten Derivaten; Homocystein und seinen N-alkylierten, N-acylierten oder C-alkylierten Derivaten; und Peptiden, welche sie umfassen, wie zum Beispiel reduziertes Glutathion;
- Cysteamin oder seinen N-alkylierten oder N-acylierten Derivaten;
- Thioglycolsäure oder einem von ihren Derivaten, Thiomilchsäure oder einem von ihren Derivaten oder Mercaptopropionsäure oder einem von ihren Derivaten.

5. Verwendung wie in einem der vorhergehenden Ansprüche beansprucht, wobei der nukleophile Wirkstoff aus Cystein oder einem von seinen N-alkylierten, N-acylierten oder C-alkylierten Derivaten; Homocystein und seinen N-alkylierten, N-acylierten oder C-alkylierten Derivaten; und Peptiden, welche sie umfassen, wie zum Beispiel reduziertes Glutathion, ausgewählt ist.

6. Verwendung wie in einem der vorhergehenden Ansprüche beansprucht, wobei der Wirkstoff mit einem pKa über 11 aus Guanidin oder einem von seinen Derivaten; Imidazol oder einer Verbindung, umfassend eine Imidazolylgruppe; oder Pyrrol oder einer Verbindung, umfassend eine Pyrrolylgruppe, ausgewählt ist.

7. Verwendung wie in einem der vorhergehenden Ansprüche beansprucht, wobei der Wirkstoff mit einem pKa über 11 aus Guanidin und seinen Derivaten ausgewählt ist.

8. Verwendung wie in einem der vorhergehenden Ansprüche beansprucht, wobei der Wirkstoff mit einem pKa über 11 das Guanidiniumion, stärker besonders verwendet in der Form von Guanidincarbonat, Guanidinhydroxid oder Guanidinthiocyanat, ist.

9. Verwendung wie in einem der vorhergehenden Ansprüche beansprucht, wobei der nukleophile Wirkstoff ein Thiolderivat, ausgewählt insbesondere aus Cystein und seinen N-alkylierten, N-acylierten und C-alkylierten Derivaten, ist und der Wirkstoff mit einem pKa über 11 Guanidin oder eines von seinen Derivaten ist.

10. Verwendung wie in einem der vorhergehenden Ansprüche beansprucht, wobei der nukleophile Wirkstoff N-Acetyl-L-cystein ist und der Wirkstoff mit einem pKa über 11 das Guanidiniumion, insbesondere in der Form von Guanidincarbonat, ist.

11. Verwendung wie in einem der vorhergehenden Ansprüche beansprucht, wobei der nukleophile Wirkstoff und der Wirkstoff mit einem pKa über 11 in einem Molverhältnis von nukleophilem Wirkstoff/Wirkstoff mit einem pKa über 11 in einem Bereich von 10 bis 1, insbesondere von 5 bis 1 und stärker besonders von ungefähr 1 verwendet werden.

12. Kosmetisches Verfahren zum Behandeln von Anzeichen von Alterung der Haut im Zusammenhang mit Glykierung, insbesondere beabsichtigt zum Wiederherstellen von Geschmeidigkeit von glykierter Haut, umfassend mindestens die Schritte, welche bestehen aus:
(i) Inkontaktbringen der zu behandelnden Haut mit mindestens einem nukleophilen Wirkstoff; und
(ii) Inkontaktbringen der zu behandelnden Haut mit mindestens einem stickstoffhaltigen Wirkstoff mit einem pKa über 11, welcher verschieden von dem nukleophilen Wirkstoff ist;
wobei es möglich ist, dass die Schritte (i) und (ii) aufeinanderfolgend in der chronologischen Reihenfolge (i) und dann (ii) oder in der umgekehrten Reihenfolge (ii) und dann (i), oder gleichzeitig durchgeführt werden, und die Anzeichen aus einer Gruppe ausgewählt sind, welche einen Verlust von Elastizität der Haut, einen Verlust von Geschmeidigkeit der Haut, eine Farbänderung der Haut, ein Elastose-Phänomen der Haut und ein Orangehaut-Phänomen der Haut umfasst.

13. Verfahren wie in Anspruch 12 beansprucht, **dadurch gekennzeichnet, dass** es Inkontaktbringen der Haut mit einer Zusammensetzung, welche erhalten wird durch Rezepturmischen von mindestens einer Zusammensetzung, umfassend den nukleophilen Wirkstoff, und mindestens einer Zusammensetzung, umfassend den Wirkstoff mit einem pKa über 11, umfasst.

14. Verfahren wie in Anspruch 12 oder 13 beansprucht, **dadurch gekennzeichnet, dass** es die Aufbringung auf die und/oder die Injektion in die Dicke der Haut von einer oder mehr Zusammensetzungen, umfassend den nukelophilen Wirkstoff und/oder den Wirkstoff mit einem pKa über 11, umfasst.

15. Verfahren wie in einem der Ansprüche 12 bis 14 beansprucht, **dadurch gekennzeichnet, dass** der nukleophile Wirkstoff und der Wirkstoff mit einem pKa über 11 wie in einem der Ansprüche 3 bis 11 definiert sind.

16. Verfahren wie in einem der Ansprüche 12 bis 15 beansprucht, wobei der Vorgang, während welchem die Haut mit dem nukelophilen Wirkstoff und/oder dem Wirkstoff mit einem pKa über 11 in Kontakt gebracht wird, begleitet wird von der Verwendung von einem oder mehr Mitteln zum Fördern der Penetration des Wirkstoffes oder der Wirkstoffe in die Haut, insbesondere Verbindungen, welche die Penetration in die Haut fördern, wie einem Lösungsmittel, grenzflächenaktiven Mittel oder Hydrotrop, einer mechanischen Handlung, wie einem Mikropeeling, oder einer energetischen Handlung, wie der Anwendung von Wärme, elektrischen Strömen, elektromagnetischen Wellen, Mikrowellen oder Ultraschall.

17. Kosmetischer Kit, umfassend in getrennten Behältern mindestens eine erste Zusammensetzung, umfassend einen nukleophilen Wirkstoff, mindestens eine zweite Zusammensetzung, umfassend einen stickstoffhaltigen Wirkstoff mit einem pKa über 11, insbesondere wie in einem der Ansprüche 3 bis 11 definiert, und mindestens ein Mittel zum Aufbringen einer Zusammensetzung auf die Haut oder ein Mittel zum Injizieren einer Zusammensetzung in die Dicke der Haut.

## Revendications

1. Utilisation cosmétique d'une association d'au moins un agent nucléophile et d'au moins un agent azoté de pKa supérieur à 11, distinct dudit agent nucléophile, pour traiter les signes de vieillissement et de photovieillissement de la peau liés à la glycation, lesdits signes étant choisis dans le groupe comprenant une perte de souplesse de la peau, un changement de couleur de la peau, un aspect élastosique de la peau et un aspect de peau d'orange de la peau.

2. Utilisation cosmétique selon la revendication 1 pour assouplir une peau glyquée.

3. Utilisation selon l'une ou l'autre des revendications précédentes, dans laquelle ledit agent nucléophile est choisi parmi les composés possédant un groupe fonctionnel nucléophile choisi parmi les groupes fonctionnels suivants :
- thiolate ;
- halogénure, en particulier iodure, bromure, chlorure ou fluorure et les groupes fonctionnels de type halogénure oxydé comme par exemple hypoiodite ;
- cyanate et ses dérivés, comme par exemple isocyanate ou isothiocyanate ;
- sulfite et autres groupes fonctionnels de type soufre oxydé, comme par exemple bisulfite ou hydrogénosulfite, thiosulfate et tétrathionate ; et
- phosphite, phosphine et ses dérivés, en particulier triarylphosphine, trialkylphosphine comme par exemple triacide phosphinetripropionique ou triphénylphosphine.

4. Utilisation selon l'une quelconque des revendications précédentes, dans laquelle ledit agent nucléophile est un dérivé thiol, en particulier choisi parmi :
- la cystéine et ses dérivés N-alkylés, N-acylés ou C-alkylés ; l'homocystéine et ses dérivés N-alkylés, N-acylés ou C-alkylés ; des peptides les contenant comme par exemple le glutathion réduit ;
- la cystéamine ou ses dérivés N-alkylés ou N-acylés ;
- l'acide thioglycolique ou l'un de ses dérivés, l'acide thiolactique ou l'un de ses dérivés, ou l'acide mercaptopropionique ou l'un de ses dérivés.

5. Utilisation selon l'une quelconque des revendications précédentes, dans laquelle ledit agent nucléophile est choisi parmi la cystéine ou l'un de ses dérivés N-alkylés, N-acylés ou C-alkylés ; l'homocystéine et ses dérivés N-alkylés, N-acylés ou C-alkylés ; et des peptides les contenant comme par exemple le glutathion réduit.

6. Utilisation selon l'une quelconque des revendications précédentes, dans laquelle ledit agent de pKa supérieur à 11 est choisi parmi la guanidine ou l'un de ses dérivés ; l'imidazole ou un composé comportant un groupement imidazolyle ; le pyrrole ou un composé comportant un groupement pyrrolyle.

7. Utilisation selon l'une quelconque des revendications précédentes, dans laquelle ledit agent de pKa supérieur à 11 est choisi parmi la guanidine et ses dérivés.

8. Utilisation selon l'une quelconque des revendications précédentes, dans laquelle ledit agent de pKa supérieur à 11 est l'ion guanidinium, plus particulièrement employé sous la forme de carbonate de guanidine, d'hydroxyde de guanidine ou de thiocyanate de guanidine.

9. Utilisation selon l'une quelconque des revendications précédentes, dans laquelle ledit agent nucléophile est un dérivé thiol, en particulier choisi parmi la cystéine et ses dérivés N-alkylés, N-acylés et C-alkylés ; et ledit agent de pKa supérieur à 11 est la guanidine ou l'un de ses dérivés.

10. Utilisation selon l'une quelconque des revendications précédentes, dans laquelle ledit agent nucléophile est la N-acétyl-L-cystéine et ledit agent de pKa supérieur à 11 est l'ion guanidinium, en particulier sous forme de carbonate de guanidine.

11. Utilisation selon l'une quelconque des revendications précédentes, dans laquelle ledit agent nucléophile et ledit agent de pKa supérieur à 11 sont employés en un rapport molaire agent nucléophile/agent de pKa supérieur à 11, allant de 10 à 1, en particulier de 5 à 1 et plus particulièrement d'environ 1.

12. Procédé cosmétique pour traiter les signes de vieillissement de la peau liés à la glycation, en particulier destiné à assouplir une peau glyquée, comprenant au moins les étapes consistant à :
(i) mettre en contact la peau à traiter avec au moins un agent nucléophile ; et
(ii) mettre en contact la peau à traiter avec au moins agent azoté de pKa supérieur à 11, distinct dudit agent nucléophile ;
les étapes (i) et (ii) pouvant être mises en oeuvre consécutivement dans l'ordre chronologique (i) puis (ii) ou dans l'ordre inverse (ii) puis (i), ou simultanément, et lesdits signes étant choisis dans le groupe comprenant une perte de souplesse de la peau, un changement de couleur de la peau, un aspect élastosique de la peau et un aspect de peau d'orange de la peau.

13. Procédé selon la revendication 12, **caractérisé en ce qu'**il comprend la mise en contact avec la peau d'une composition obtenue par mélange extemporané d'au moins une composition comprenant ledit agent nucléophile et d'au moins une composition comprenant ledit agent de pKa supérieur à 11.

14. Procédé selon la revendication 12 ou 13, **caractérisé en ce qu'**il comprend l'application et/ou l'injection dans l'épaisseur de la peau d'une ou plusieurs compositions comprenant ledit agent nucléophile et/ou ledit agent de pKa supérieur à 11.

15. Procédé selon l'une quelconque des revendications 12 à 14, **caractérisé en ce que** ledit agent nucléophile et ledit agent de pKa supérieur à 11 sont tels que définis dans l'une quelconque des revendications 3 à 11.

16. Procédé selon l'une quelconque des revendications 12 à 15, dans lequel l'opération dans laquelle la peau est portée en contact avec ledit agent nucléophile et/ou ledit agent de pKa supérieur à 11 s'accompagne de l'emploi d'un ou plusieurs moyens pour favoriser la pénétration dudit agent ou desdits agents dans la peau, en particulier de composés favorisant la pénétration dans la peau tels qu'un solvant, tensioactif ou hydrotrope, une action mécanique telle qu'un micropeeling, ou une action énergétique telle que l'application de chaleur, de courants électriques, d'ondes électromagnétiques, de microondes ou d'ultrasons.

17. Kit cosmétique comprenant, dans des récipients séparés, au moins une première composition comprenant un agent nucléophile, au moins une deuxième composition comprenant un agent azoté de pKa supérieur à 11, en particulier comme défini dans l'une quelconque des revendications 3 à 11, et au moins un moyen d'application ou d'injection d'une composition dans l'épaisseur de la peau.
